# FASCICULE DE BREVET EUROPEEN

(11) **EP 1 641 463 B1**
(45) Date de publication et mention de la délivrance du brevet: **11.03.2009**
(21) Numéro de dépôt: 04763072.8
(22) Date de dépôt: 18.06.2004
(51) Int. Cl.: A61K 31/573, A61K 47/14, A61K 47/24, A61K 47/44

(54) **COMPOSITIONS SOUS FORME DE SPRAY COMPRENANT UN ACTIF PHARMACEUTIQUE, AU MOINS UN SILICONE VOLATILE ET UNE PHASE HUILEUSE NON VOLATILE**
ZUSAMMENSETZUNGEN IN FORM EINES SPRAYS MIT EINEM PHARMAZEUTISCHEN MITTEL, MINDESTENS EINEM FLÜCHTIGEN SILIKON UND EINER NICHTFLÜCHTIGEN ÖLIGEN PHASE
COMPOSITIONS IN THE FORM OF A SPRAY COMPRISING A PHARMACEUTICAL AGENT AT LEAST ONE VOLATILE SILICONE AND A NON-VOLATILE OILY PHASE

(30) Priorité: 23.06.2003 FR 0307551
(43) Date de publication de la demande: 05.04.2006
(73) Titulaire: Galderma Research & Development, 06410 Biot (FR)
(72) Inventeur: MALLARD, Claire, F-06250 Mougins le Haut (FR); PITRE, Franck, F-77600 Bussy Saint Georges (FR); FREDON, Laurent, F-06330 Roquefort Les Pins (FR)
(74) Mandataire: Allab, Myriam
(86) Numéro de dépôt international: PCT/EP2004/007203
(87) Numéro de publication internationale: WO 2004/112798

(56) Documents cités:
- EP-A- 0 844 001
- EP-A- 0 966 972
- WO-A-00/37027
- WO-A-01/87344
- US-A- 4 678 663
- US-A- 4 889 845
- US-B1- 6 325 990
- US-B1- 6 512 072
- US-B2- 6 538 039
- PATENT ABSTRACTS OF JAPAN vol. 0143, no. 90 (C-0751), 23 août 1990 (1990-08-23) & JP 2 145512 A (SHIN ETSU CHEM CO LTD), 5 juin 1990 (1990-06-05)
- "Temovate-Product information"[Online] juin 2000 (2000-06), XP002262263 Extrait de l'Internet: URL:http://us.gsk.com/products/assets/us_t emovate.pdf> [extrait le 2003-11-19]

## Description

L'invention se rapporte à une composition comprenant un actif pharmaceutique, au moins un silicone volatile et une phase huileuse, non volatile dans un milieu physiologiquement acceptable, à son procédé de préparation et à son utilisation en cosmétique et en dermatologie, la composition permettant d'obtenir une bonne pénétration de l'actif à travers les couches cutanées.

Dans le domaine de la dermatologie et de la formulation de compositions pharmaceutiques, l'homme du métier est amené à chercher des compositions qui permettent de libérer l'actif et de favoriser sa pénétration à travers les couches cutanées afin d'en améliorer son efficacité. Le produit doit en outre présenter une bonne cosméticité et être préférentiellement non irritant.

Il existe actuellement de nombreuses compositions topiques comprenant un agent actif et permettant de favoriser sa pénétration dans la peau grâce à la présence notamment d'une forte teneur en glycol pro-pénétrant. Ces compositions sont formulées sous formes d'émulsions à forte teneur en phase grasse que l'on appelle communément "lipocremes", sous formes de compositions anhydres que l'on appelle "onguents", sous forme de compositions fluides à forte teneur en solvants volatiles, tels que l'éthanol ou l'isopropanol, destinées à une applications sur le cuir chevelu, appelées également "lotions capillaires", ou encore sous forme d'émulsions H/E visqueuses, que l'on appelle aussi "crèmes H/E".

On connaît par exemple des crèmes H/E comprenant un corticoïde et un fort pourcentage de propylène glycol (47,5 %) commercialisées sous la marque TEMOVATE^{®} par la société GLAXOSMITHKLINE. La stabilisation d'une formulation comprenant un tel pourcentage de glycol rend nécessaire l'emploi dans l'émulsion d'agents émulsifiants et stabilisants de type glycéryl stéarate ou PEG 100 stéarate ou encore d'agents stabilisants ou facteurs de consistance de type cire blanche ou alcool cétostéarylique qui conduisent à la formation d'une crème visqueuse, c'est à dire dont la viscosité est supérieure à 10 Pa.s (10000 centipoises, mesurée avec un appareil Brookfield modèle LVDV II + mobile n° 4, à une vitesse de 30 tours/min pendant 30 secondes et à une température de 25 °C +/- 3 °C). Cette viscosité confère donc au produit une difficulté d'application. Ces compositions invention.

Pour faciliter l'application de compositions topiques comprenant un fort pourcentage de glycol pro-pénétrant, la demanderesse a réalisé, et protégé par la demande EP832647, une lotion, formulation stable de type émulsion H/E, dont la viscosité est intermédiaire entre les lotions capillaires trop fluides et d'un usage trop limité, et les crèmes H/E trop visqueuses et présentant un coté gras et collant, tout en conservant les propriétés pro-pénétrantes du glycol. Ces formules présentent effectivement une bonne pénétration de l'actif mais comprennent toujours un pourcentage élevé de glycol qui peut donc induire un effet collant ou des problèmes de tolérance conduisant à une acceptabilité moyenne du produit par le patient.

L'homme du métier connaît par ailleurs des formulations contenant des composés siliconés conduisant à des compositions agréables d'utilisation. Ainsi, dans les brevets US 6,538,039 et US 6,325,990, une nouvelle formulation d'actif pour une administration transdermique a été mise au point comprenant des composés siliconés afin de déposer un film à la surface de la peau. Dans ces demandes également, le passage transdermique est facilité par la présence obligatoire de promoteur d'absorption que sont, entre autres composés cités, les glycols.
Dans la demande WO00/37027, des compositions comprenant de la nadifloxaxcine sont décrites. Dans le brevet US4889845, des compositions stables comprenant des prostaglandines de type E sont décrites.

Dans la demande de brevet EP0966972, les compositions décrites peuvent être formulées sous forme de spray et comprennent un composé actif, une gomme silicone et un excipient pharmaceutiquement acceptable. Le problème que se propose de résoudre l'invention décrite dans EP0966972 est de déposer un film substantif à la surface de la peau, problème résolu grâce à la présence de la gomme de silicone.

Le problème que se propose de résoudre ici la présente invention, est de concevoir une composition permettant d'améliorer la pénétration de l'actif pharmaceutique, et sa rapidité de pénétration dans le temps afin d'en améliorer son efficacité thérapeutique, tout en évitant la présence de forte teneur en glycol. La composition selon invention doit également présenter une facilité d'utilisation et une cosméticité acceptable pour une application sur toutes les zones du corps pouvant être touchées par la pathologie.

Les deux demandes EP0966972 et US 6,538,039 représentent l'art antérieur le plus proche de la présente invention, compte tenu de la composition des formulations décrites. Mais à la lecture de cet art antérieur, rien ne pouvait inciter l'homme du métier à choisir la composition selon l'invention afin d'obtenir une bonne pénétration de l'actif incorporé dans les couches cutanées.

En effet, la demanderesse a trouvé de manière surprenante que la composition pulvérisable comprenant, dans un véhicule alcoolique pharmaceutiquement acceptable et solubilisant de l'actif
a) une quantité thérapeutiquement efficace de 17-propionate de clobétasol
b) au moins un silicone volatile,
c) une phase huileuse non volatile,
conduisait à une amélioration de la pénétration de l'actif.

La composition de la présente invention, tout en permettant une bonne pénétration des principes actifs, présente également une très bonne acceptabilité et tolérance auprès des patients, comme décrit dans les exemples 8 et 9 de la présente invention. Il s'avère donc que la composition selon l'invention est particulièrement adaptée au traitement des affections dermatologiques et plus particulièrement bien adaptée pour le traitement du psoriasis.

L'invention concerne donc une composition pulvérisable comprenant, dans un véhicule alcoolique pharmaceutiquement acceptable et solubilisant de l'actif
a) une quantité thérapeutiquement efficace de 17-propionate de clobétasol,
b) au moins un silicone volatile,
c) une phase huileuse non volatile.

L'actif selon l'invention peut être utilisé seul ou en association.

Avantageusement, la composition selon l'invention comprend entre 0,0001 et 20 % en poids par rapport au poids total de la composition d'un agent actif, de préférence entre 0,025 et 15 % en poids, et plus préférentiellement entre 0.01 et 5 % en poids.

La composition selon l'invention, comprendra le 17 propionate de clobétasol, de préférence à une concentration inférieure à 2 % et de préférence, comprise entre 0,01 et 2 % en poids, plus préférentiellement entre 0,025 et 0,1 % en poids. L'actif pharmaceutique préféré selon l'invention est le 17-propionate de clobétasol utilisé à la concentration de 0.05 % en poids.

Par silicone volatile selon l'invention, on entend des composés polyorganosiloxanes, pouvant être cycliques ou linéaires, ayant une pression mesurable dans des conditions ambiantes.

Les silicones volatiles cycliques selon l'invention, sont les polydiméthylcyclosiloxanes, à savoir des composés de formule : 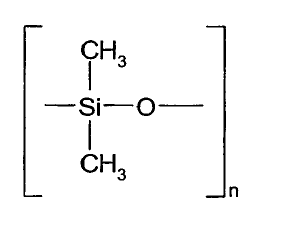 avec n compris entre 3 et 6 en moyenne et de préférence n=4 ou n=5, généralement connu sous le nom de cyclométhicones.

Les silicones volatiles linéaires selon l'invention sont des polysiloxanes linéaires de bas poids moléculaire tels l'héxaméthyldisiloxane ou les diméthicones de bas poids moléculaire. Les silicones volatiles linéaires ont généralement une viscosité inférieure à environ 5 centistokes à 25°C, alors que les silicones volatiles cycliques ont une viscosité inférieure à environ 10 centistokes à 25°C.

Les silicones volatiles préférés selon l'invention sont les siloxanes linéaires et plus préférentiellement l'hexaméthyldisiloxane. On peut citer à titre d'exemple le produit commercialisé par la société DOW CORNING, le DC Fluid 0.65cSt.

Avantageusement, la composition selon l'invention comprend entre 25 et 95 % en poids par rapport au poids total de la composition du silicone volatile, de préférence entre 40 et 80 % en poids, et plus préférentiellement entre 55 et 65 % en poids.

Par phase huileuse, non volatile selon l'invention, on entend une variété d'huile non volatile convenant pour une composition pharmaceutique ou cosmétique. Les huiles non volatiles ont généralement une viscosité supérieure à environ 10 centipoises à 25°C, et peuvent atteindre une viscosité allant jusqu'à 1 000 000 centipoises à 25°C. La phase huileuse non volatile peut être composée d'une large variété d'huiles synthétiques ou naturelles, silicones ou organiques, dont une liste non exhaustive est donné à titre indicatif.

### (a) les Esters

Des exemples d'huile non volatile utilisable selon l'invention comprennent les esters de formule RCO-OR' avec R et R', identiques ou différents, représentant une chaine, linéaire ou ramifiée, d'un alkyl, alkényl, alkoxycarbonylalkyl, ou alkoxycarbonyloxyalkyl ayant de 1 à 25 atomes de carbone, de préférence de 4 à 20 atomes de carbone. Des exemples de tels esters incluent l'isononaote d'isotricédyle, le diheptanoate de PEG-4, le néopentanoate d'isostéaryle, le néopentanoate de tridécyle, l'octanoate de cétyle, le palmitate de cétyle, le ricinoléate de cétyle, le stéarate de cétyle, le myristate de cétyle, le dicaprylate/caprate de coco, l'isostéarate de décyle, l'oléate d'isodécyle, le néopentanoate d'isodécyle, le néopentanoate d'isohéxyle, le palmitate d'octyle, le malate de dioctyle, l'octanoate de tridécyle, le myristate de myristyle, l'octododécanol.

### (b) les Esters glycéryles d'acides gras

L'huile peut également comprendre également les esters gras d'acides gras naturels, ou les triglycérides de source animale ou végétale. De tels exemples incluent, l'huile de castor, l'huile de lanoline, le citrate de triisocétyle, les triglycérides ayant de 10 à 18 atomes de carbone, les triglycérides capryliques/capriques, l'huile de noix de coco, l'huile de mais, l'huile de coton, l'huile de lin, l'huile de vison, l'huile d'olive, l'huile de palme, le beurre d'illipé, l'huile de colza, l'huile de soja, l'huile de tournesol, l'huile de noix et équivalent.

### (c) Les glycérydes d'acides gras

Les huiles qui conviennent également sont les esters glycéryles synthétiques ou semi-synthétiques, comme les mono-, di-, triglycérides d'acides gras, qui sont des huiles ou des graisses naturelles modifiées, pas exemple, le stearate de glycéryle, le dioléate de glycéryle, le distéarate de glycéryle, le trioctanoate de glycéryle, le distearate de glycéryle, le linoléate de glycéryle, le myristate de glycéryle, l'isostéarate de glycéryle, les huiles de castor PEG, les oléates de glycéryle PEG, les stearates de glycéryle PEG, et équivalent.

### (d) Les hydrocarbones non volatiles

Conviennent également très bien à la composition selon l'invention en tant que phase huileuse non volatile, les hydrocarbones non volatiles, telles que les paraffines, les isoparaffines, les huiles minérales, et équivalent.

### (e) Les esters de Guerbet

Les esters de Guerbet sont des esters résultant de la réaction d'un alcool de Guerbet de formule générale : 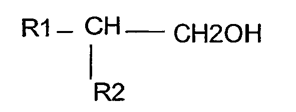 et d'un acide carboxylique de formule générale R3-COOH ou HOOC-R3-COOH,
avec R1 et R2, identiques ou différents représentent un alkyl ayant de 4 à 20 atomes de carbone, R3 représente un radical gras substitué ou non, tel qu'une chaîne alkyle ou alkylène, linéaire ou ramifiée, saturée ou insaturée, ayant de 1 à 50 atomes de carbone, un phényle, pouvant être substitué par un halogène, un hydroxyle, un carboxyle, ou un alkylcarbonylhydroxy.

### (f) Les huiles siliconées

Les huiles siliconées utilisables selon l'invention pour la constitution de la phase non volatile sont des composés polyorganosiloxanes, ayant une pression mesurable dans des conditions ambiante et une viscosité strictement supérieure à 10 centistokes. Les silicones non volatiles utilisables selon l'invention sont les composés de formule 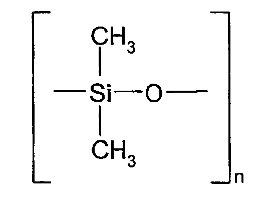 avec n strictement supérieur à 6.

La phase huileuse, non volatile préférée selon l'invention est l'huile de paraffine.

Avantageusement, la composition selon l'invention comprend entre 1 et 50 % en poids par rapport au poids total de la composition de phase huileuse, non volatile, de préférence entre 5 et 30 % en poids, et plus préférentiellement entre 5 et 15 % en poids.

Ainsi une composition préférée selon l'invention sera une composition pulvérisable comprenant :
a) Entre 0,0001 et 20 % en poids de l'actif pharmaceutique,
b) Entre 25 et 95 % en poids de silicone volatile,
c) Entre 1 et 50% en poids de phase huileuse non volatile.

Selon un mode préféré de composition selon l'invention, la composition comprend également une gomme de silicone. La demanderesse a, en effet, découvert de manière surprenante qu'une composition comprenant une gomme silicone dans les concentrations définies ci-après présente une pénétration plus rapide de l'actif à travers les différentes couches cutanées.

Par gommes de silicone, on entend les gommes de silicones connues par l'homme de l'art et notamment celles décrites dans la demande de brevet EP 0 966 972 incorporée ici par référence. Selon ce mode préféré de composition selon l'invention, la gomme de silicone est introduite à la concentration comprise entre 0,001 et 3 % en poids, de préférence entre 0,01 et 1 % en poids. Dow Corning propose un produit commercial vendu sous le nom de DC Silmogen Carrier qui se compose de 99% d'hexaméthyldisiloxane et de 1 % de gomme de silicone, produit qui pourra avantageusement être utilisé dans une des compositions selon l'invention.

Le véhicule pharmaceutiquement acceptable selon l'invention doit être choisi de telle manière que les propriétés avantageuses attachées intrinsèquement à la présente invention ne soient pas ou substantiellement pas altérées par l'addition envisagée. De façon préférée, le véhicule utilisé selon l'invention est choisi de façon à être un solubilisant de l'actif. Le véhicule solubilisant de l'actif peut être composé d'un seul excipient tel un solvant, ou d'un mélange d'excipients tels ceux utilisés pour la formulation d'une émulsion. A titre d'exemples non limitatifs d'excipients pouvant être utilisés seuls ou en mélange, on peut citer, l'eau, les solvants, les diluants, tout excipient utilisable pour la formulation d'une émulsion, d'un lait, d'un gel, d'un onguent, d'une composition moussante. Ces excipients sont des composés couramment utilisés dans la formulation de composition pharmaceutique. De façon préférentielle, les excipients solubilisant de l'actif selon l'invention sont l'eau, les alcools, les polyols, les éthers, les esters, les aldéhydes, les cétones, les acides et alcools gras, les esters gras. Plus préférentiellement, l'excipient utilisé sera un alcool. Par alcool selon l'invention, on entend, les alcools aliphatiques, linéaires ou ramifiés, tel l'éthanol, le propanol, l'isopropanol.

Dans un mode préféré selon l'invention, le véhicule utilisé sera donc alcoolique.
Par véhicule alcoolique selon l'invention, on entend un véhicule comprenant au moins 15% d'alcool, et préférentiellement au moins 25% d'éthanol.

En particulier, la composition selon l'invention telle que décrite précédemment sera telle qu'elle contient :
a) 0.05% de 17-propionate de clobétasol
b) 60% d' hexaméthyldisiloxane,
c) 10% d'huile de paraffine,
d) 29.95% d'éthanol.

Plus particulièrement, la composition selon l'invention sera telle qu'elle comprend :
a) 0.05% de 17-propionate de clobétasol
b) 59.4% d' hexaméthyldisiloxane,
c) 0.6% de gomme de silicone,
d) 10% d'huile de paraffine,
e) 29.95% d'éthanol.

La composition pharmaceutique selon l'invention pourra en outre contenir des additifs inertes ou des combinaisons de ces additifs, tels que
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des agents propénétrants,
- des antioxydants,
- et des polymères synthétiques.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

La composition selon l'invention est plus particulièrement destinée au traitement de la peau et des muqueuses et peut se présenter sous forme d'onguents, de crèmes, de laits, de pommades, de poudres, de tampons imbibés, de syndets, de solutions, de gels, de sprays, de mousses, de suspensions, de lotions, de sticks, de shampoings, de pledgets, ou de base lavante. Elle peut également se présenter sous forme de suspensions de microsphères ou nanosphères ou vésicules lipidiques ou polymériques ou de patchs polymériques et d'hydrogels permettant une libération contrôlée. Cette composition par voie topique peut se présenter sous forme anhydre, sous forme aqueuse ou sous la forme d'une émulsion.

La composition selon l'invention présentant une pénétration améliorée est administrée de préférence sous la forme d'une composition pulvérisable. Afin d'être pulvérisable, les compositions selon l'invention présenteront préférentiellement une viscosité inférieure à 50 centistokes et plus préférentiellement inférieure à 10 centistokes.
Le spray peut être obtenu par des moyens conventionnels de formulation connus de l'homme du métier. Par exemple, la composition peut être pulvérisée par un pulvérisateur mécanique qui pompe la composition au sein d'un récipient, flacon ou équivalent. La composition passe à travers une buse qui peut être dirigée directement à l'endroit désiré de l'application. La buse peut être choisie de façon à appliquer la composition sous forme d'une vaporisation ou d'un jet de gouttelette, selon les techniques connues de l'homme de l'art. Selon l'actif pharmaceutique choisi, le mécanisme de pulvérisation doit être capable de délivrer toujours la même quantité d'actif. Les mécanismes permettant de contrôler la quantité de composition à délivrer par le spray sont également connues de l'homme de l'art. Par exemple, la quantité de gaz propulseur peut être calculée de façon à propulser l'exacte quantité de produit désirée.

De préférence pour la composition selon l'invention, on utilisera un flacon vaporisateur doseur dont les caractéristiques de surface d'application et de doses sont contrôlées et reproductibles. Par exemple, le vaporisateur utilisé est constitué d'un flacon équipé d'une valve doseuse de 25µl.

La présente invention a également pour objet l'utilisation d'une composition selon l'invention pour la fabrication d'un médicament destiné au traitement :
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle,
- des ichtyoses, des états ichtyosiformes, de la maladie de Darrier, des kératodermies palmoplantaires, des leucoplasies et des états leucoplasiformes, du lichen cutané ou muqueux (buccal), '
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma, l'atopie respiratoire ou l'hypertrophie gingivale,
- des proliférations dermiques ou épidermiques bénignes ou malignes, d'origine virale ou non, notamment les verrues vulgaires, les verrues planes l'épidermodysplasie verruciforme, les papillomatoses orales ou florides, le lymphome T,
- des proliférations pouvant être induites par les ultra-violets notamment des épithélioma baso et spinocellulaires,
- des lésions précancéreuses cutanées notamment les kératoacanthomes,
- des dermatoses immunes notamment le lupus érythémateux,
- des maladies immunes bulleuses,
- des maladies du collagène notamment la sclérodermie,
- des affections dermatologiques ou générales à composante immunologique,
- de désordres cutanés dus à une exposition aux rayonnements U.V, du vieillissement de la peau, photo-induit ou chronologique ou des pigmentations et des kératoses actiniques, ou toutes pathologies associées au vieillissement chronologique ou actinique notamment la xérose,
- des troubles de la fonction sébacée notamment l'hyperséborrhée de l'acné, la séborrhée simple ou la dermite séborrhéique,
- des troubles de la cicatrisation ou des vergetures,
- des désordres de la pigmentation, tel l'hyperpigmentation, le mélasma, l'hypopigmentation ou le vitiligo,
- des affections du métabolisme des lipides, tel l'obésité, l'hyperlipidémie, le diabète non insulino-dépendant ou le syndrome X,
- des affections inflammatoires telles que l'arthrite,
- des états cancéreux ou précancéreux,
- de l'alopécie de différentes origines, notamment l'alopécie due à la chimiothérapie ou aux rayonnements,
- des troubles du systèmes immunitaire, tel l'asthme, le diabète sucré de type 1, la sclérose en plaque, ou autres disfonctionnements sélectifs du système immunitaire, ou
- des affections du système cardiovasculaire telles que l'artériosclérose ou l'hypertension.

Dans un mode préféré d'utilisation de la composition, celle-ci contiendra 0.05% de 17-propionate de clobétasol et sera utilisée pour la fabrication d'un médicament destiné à traiter le psoriasis.

L'invention concerne également un procédé d'amélioration de la pénétration d'un actif, caractérisé en ce que l'on applique sur la peau une composition comprenant, dans un véhicule alcoolique pharmaceutiquement acceptable :
a) une quantité thérapeutiquement efficace d'un actif pharmaceutique,
b) au moins un silicone volatile,
c) une phase huileuse non volatile,
ladite composition étant appliquée par pulvérisation.

De préférence, le procédé sera tel que l'actif est le 17-propionate de clobetasol, le silicone volatile est l'hexamethyldisiloxane et la phase huileuse non volatile est l'huile de paraffine.

Dans une variante de mise en oeuvre, le procédé sera tel que la composition comprend également une gomme de silicone.

En effet, la demanderesse a découvert de manière surprenante que la pénétration d'un actif à travers la peau était améliorée par la composition selon l'invention. Par amélioration de la pénétration dans la peau, on entend une augmentation significative de la pénétration dans la peau d'au moins un facteur 2 par rapport aux formulations connues sur le marché. La mesure de la pénétration de l'actif est effectuée selon le protocole décrit à l'exemple 7

Les exemples suivants montrent de façon non exhaustive des exemples de formulation de la composition selon l'invention et des résultats de pénétration dans la peau ainsi que des résultats d'acceptabilité et de tolérance de la composition selon l'invention comparé à des formules existantes.

### Exemple 1 : composition

La formulation est obtenue en mélangeant les différents composés cités ci-dessous jusqu'à l'obtention d'une solution homogène et limpide.

| Ingredients | Fonction | Spray A |
|---|---|---|
| 17-propionate de clobétasol | Actif | 0.05% |
| Hexaméthyldisiloxane | Silicone volatile | 60.0% |
| Huile de Paraffine | Phase huileuse non | 10.0% |
| | volatile | |
| Ethanol absolu | Solvant : excipient | qsp 100% |

### Exemple 2 : composition

Le mode opératoire utilisé est le même que celui de l'exemple 1.

| Ingredients | Fonction | Spray B |
|---|---|---|
| 17-propionate de clobétasol | Actif | 0.05% |
| Hexaméthyldisiloxane | Silicone volatile | 59.4% |
| Gomme silicone | Gomme silicone | 0.6% |
| Huile de Paraffine | Phase huileuse non | 10.0% |
| | volatile | |
| Ethanol absolu | Solvant | qsp 100% |

### Exemple 3 : composition

Le mode opératoire utilisé est le même que celui de l'exemple 1.

| Ingredients | Fonction | Spray C |
|---|---|---|
| 17-propionate de clobétasol | Actif | 0.05% |
| Hexaméthyldisiloxane | Silicone volatile | 59.4% |
| Gomme silicone | Gomme silicone | 0.6% |
| Huile de Paraffine | Phase huileuse | 10.0% |
| | non volatile | |
| Acide Oleic | Pro pénétrant | 5.0% |
| Butylhydroxytoluène (BHT) | Antioxidant | 0.05% |
| Ethanol absolu | Solvant | qsp 100% |

### Exemple 4 : composition

Le mode opératoire utilisé est le même que celui de l'exemple 1.

| Ingredients | Fonction | Spray D |
|---|---|---|
| Amorolfine HCI | Actif | 5.0% |

| | | |
|---|---|---|
| Hexaméthyldisiloxane | Silicone volatile | 59.4% |
| Gomme silicone | Gomme silicone | 0.6% |
| Isodécyl oléate | Phase huileuse non | 5.0% |
| | volatile | |
| Urée | Propénétrant | 5.0 |
| Ethanol absolu | Solvant | qsp 100% |

### Exemple 5 : composition

Le mode opératoire utilisé est le même que celui de l'exemple 1.

| Ingredients | Fonction | Spray E |
|---|---|---|
| Calcipotriol | Actif | 0.005% |
| Hexaméthyldisiloxane | Silicone volatile | 59.4% |
| Gomme silicone | Gomme silicone | 0.6% |
| Huile de paraffine | Phase huileuse non | 10.0% |
| | volatile | |
| Ethanol absolu | Solvant | qsp 100% |

### Exemple 6 : composition

Le mode opératoire utilisé est le même que celui de l'exemple 1.

| Ingredients | Fonction | Spray F |
|---|---|---|
| Clindamycine | Actif | 1.0% |
| Hexaméthyldisiloxane | Silicone volatile | 59.4% |
| Gomme silicone | Gomme silicone | 0.6% |
| Isopropyl myristate | Phase huileuse non | 5.0% |
| | volatile | |
| Ethanol absolu | Solvant | qsp 100% |

### Exemple 7 : Etude de la libération 1 pénétration in vitro sur peau humaine de l'actif 17-propionate de clobétasol contenu dans 4 formulations différentes dont trois sont pulvérisables.

Le premier objectif est de quantifier la pénétration cutanée de l'actif formulé dans différentes formulations *in vitro* sur peau humaine après 16 heures d'application.
Le deuxième objectif est d'évaluer l'influence de la formulation sur la cinétique de pénétration de l'actif à travers et dans la peau. Dans ce but un temps plus court d'application a été testé : 4 heures.

Formulations testées:
- Crème émolliente Temovate® à 0.05 % (w/w) de 17-propionate de clobétasol
- Spray A
- Spray B
- Spray C
La crème émolliente Temovate® est commercialisée par la société GLAXOSMITHKLINE. Les compositions exactes des trois compositions sont données dans le tableau A ci-après et correspondent aux exemples 1, 2 et 3 de la présente invention.

**TABLE A**

| Ingredients | Fonction | Spray A | Spray B | Spray C |
|---|---|---|---|---|
| 17-propionate de clobétasol | Actif | 0.05% | 0.05% | 0.05% |
| Hexaméthyldisiloxane | Silicone volatile | 60.0.% | 59.4% | 59.4% |
| Gomme silicone | Gomme silicone | / | 0.6% | 0.6% |
| Huile de Paraffine | Agent occlusif | 10.0% | 10.0% | 10.0% |
| Acide Oleic | Pro pénétrant | / | / | 5.0% |
| BHT | Antioxidant | / | / | 0.05% |
| Ethanol absolu | Solvant | qsp 100% | qsp 100% | qsp 100% |

**Conditions expérimentales :** L'absorption percutanée est évaluée grâce à des cellules de diffusion constituées de 2 compartiments séparés par la peau humaine. Les formulations ont été appliquées sans occlusion.
2 temps d'application ont été testés : 4 et 16 heures.
Les formulations ont été appliquées à raison de 20 mg de formulation pour 2 cm² (i.e. 10 microgrammes de clobétasol 17-propionate).
Pendant la durée de l'étude, le derme est en contact avec un liquide récepteur non renouvelé en fonction du temps (mode statique).
Pour chaque temps d'application, six expériences différentes ont été réalisées avec six échantillons de peau provenant de six donneurs différents.
A la fin de la période d'application, l'excès de surface est enlevé et la distribution du 17-propionate de clobétasol est quantifiée dans les différents compartiments de la peau et dans le liquide récepteur. Les concentrations de 17-propionate de clobétasol ont été quantifiées en utilisant un méthode d'HPLC/MS/MS classiquement connu de l'homme de l'art (LQ: 10 ng.mL⁻¹).
Les formules spray ont été appliquées à l'aide d'un flacon pulvérisateur équipé d'une valve doseuse de 25 µl.

Les résultats expérimentaux montrent que quelle que soit la formulation testée, l'actif est distribué principalement dans la peau (épiderme, stratum corneum inclus et derme). Les quantités totales pénétrées (Stratum corneum + Epiderme + Derme + liquide recepteur) sont :

| | | Temps d'application : 4 heures | Temps d'application : 16 heures |
|---|---|---|---|
| Temovate® crème émolliente | | | |
| Quantité totale ayant pénétrée | | | |
| | - µg | 0.52 ± 0.13 µg | 0.67 ± 0.08 µg |
| | - % dose appliquée | 5% | 7% |

| | Spray A | | |
|---|---|---|---|
| Quantité totale ayant pénétrée | | | |
| | - µg | 0.57 ± 0.23 µg | 1.35± 0.45 µg |
| | - % dose appliquée | 7% | 17% |
| | | | |

| Spray B | | | |
|---|---|---|---|
| Quantité totale ayant pénétrée | | | |
| | - µg | 0.96± 0.29 µg | 1.31 ± 0.35 µg |
| | - % dose appliquée | 11 % | 15% |
| | | | |

| Spray C | | | |
|---|---|---|---|
| Quantité totale ayant pénétrée | | | |
| | - µg | 1.01 ± 0.37 µg | 1.49 ± 0.39 µg |
| | - % dose appliquée | 10% | 14% |
| | | | |

**Résultats :** Les résultats montrent ici que le spray A présente une augmentation de la pénétration de l'actif après 16 heures d'un facteur supérieur à deux, comparée à la formule de la crème émolliente Temovate® déjà existante, bien que cette composition, formule A, selon l'invention ne comporte ni composé pro-pénétrant ni agent occlusif.

Les formules B et C, bien que comportant de la gomme silicone substantive, permettent une bonne libération de l'actif conduisant donc également à une bonne pénétration de l'actif.

La mesure effectuée au temps 4 heures permet d'évaluer l'influence des excipients sur la rapidité de pénétration de l'actif dans les différentes formes de spray. Les résultats montrent que l'ajout de gomme silicone dans les compositions Spray B et C augmente la rapidité de la pénétration comparée à la formule Spray A n'en contenant pas.

Par ailleurs, on peut noter que l'ajout au sein du spray C d'un pro-pénétrant comme l'acide oléique ne modifie pas de façon significative la pénétration de l'actif au sein d'une des formules spray selon l'invention.

A titre indicatif, les formulations de sprays selon l'invention ont également été comparées à une formulation de lotion au 17-propionate de clobétasol telle que décrite dans la demande de brevet EP 0 832 647 et comportant entre 40 et 50% de glycol pro-pénétrant. Le résultat de pénétration de l'actif au sein de cette formule est le suivant :

| | |
|---|---|
| Lotion au 17 propionate clobétasol | |
| Quantité totale ayant pénétrée* | |
| - µg | 0.60 ± 0.07 µg |
| - % dose appliquée | 12% |

| | |
|---|---|
| * la surface d'application des produits dans cette expérience est de 1cm² | |

La lotion augmente la pénétration de l'actif d'un facteur supérieur à 2 après 16 heures d'application, comparée à la formule de la crème émolliente Temovate® déjà existante. Ce résultat indique donc que les compositions selon l'invention, même en l'absence de composés pro-pénétrants, permettent d'obtenir une amélioration significative de la pénétration d'un actif comparée aux formules existantes, ou une pénétration similaire à des compositions présentant un fort pourcentage de composés pro-pénétrants.

Les formules sprays telles que décrites permettent donc de s'affranchir de l'utilisation de glycols sans diminuer la pénétration cutanée et présentent donc un intérêt supplémentaire en terme de potentiel non irritant versus les compositions comprenant de forte teneur en glycol.

### Exemple 8 : Evaluation de l'acceptabilité cosmétique des sprays selon l'invention.

Le but de cette étude a été d'évaluer les qualités cosmétiques d'une composition pulvérisable dans un test d'usage, après 5 jours d'application sur des lésions cibles de patients (15 sujets masculins ou féminins, âgés de 18 à 60 ans, et présentant au moins 3 plaques de psoriasis présentant un psoriasis léger à modéré). La composition testée ici est la formule du spray B de l'exemple 2.

De plus, cette étude devait permettre de positionner la formulation de la composition pulvérisable par rapport aux véhicules de produits déjà connus (crème et lotion).

Pour les sujets, la composition pulvérisable se démarque significativement *(p< 0.05)* des 2 autres produits pour :
- la facilité d'application,
- la rapidité du séchage,
- la rapidité de pénétration et donc
- la possibilité de s'habiller plus rapidement,
- l'absence de sensation de peau grasse et de peau collante.

Les sujets n'ont pas trouvé que la composition pulvérisable débordait trop sur la peau non malade par rapport aux 2 autres produits *(p< 0.05),* par ailleurs il leur semble moins liquide que la lotion *(p< 0.05).* Il leur parait plus pratique pour traiter les zones d'accès difficile comme le dos par rapport à la lotion *(p< 0.05)* mais pas par rapport à la crème.

En terme d'appréciation du produit, 74% des sujets ont eu un avis favorable (bon ou excellent) sur la composition pulvérisable, qui se démarque nettement de la lotion (54%), mais moins nettement de la crème (67%). Aucun patient n'a eu d'avis défavorable sur le spray contre 7% pour la lotion et 14% pour la crème.

### Exemple 9 : évaluation de la tolérance locale, du potentiel hydratant et de l'effet sur la fonction barrière et du pH de la peau

Une autre étude a été réalisée avec les sprays de compositions qualitatives et quantitatives ci-dessous dans le but d'évaluer la tolérance locale, le potentiel hydratant ainsi que l'effet sur la fonction barrière et le pH de la peau de deux véhicules pulvérisables appliqués sur les avant-bras de sujets sains.

| Ingredients | Fonction | Spray D | Spray E |
|---|---|---|---|
| Hexaméthyldisiloxane | Silicone volatile | 59.4% | 59.4% |
| Gomme silicone | Gomme silicone | 0.6% | 0.6% |
| Huile de Paraffine | Agent occlusif | / | 10.0% |
| Ethanol absolu | Solvant | qsp 100% | qsp 100% |

L'étude s'est déroulée comme suit : un test de tolérance locale d'une durée de 21 jours (plus une visite d'inclusion) a été réalisé sur 12 sujets sains, de sexe féminin ou masculin, âgés de 18 à 50 ans.
Trois zones ont été délimitées : une zone sur chaque avant bras au niveau du pli du coude et une zone non traitée sur l'avant bras droit au niveau du poignet.
Les sujets ont reçu sur les avant-bras les 2 produits à tester, selon un plan de randomisation préétabli, 1 fois par jour, tous les jours pendant 21 jours à raison de 50 µl par zone.
Les évaluations cliniques de la tolérance (érythème, desquamation, prurit et brûlure) ont été réalisées à J0, J7, J14, et J21. Une échelle de cotation de 0 à 3 a été utilisée pour évaluer l'érythème et la desquamation (0 = absent, 1 = léger, 2 = modéré et 3 = sévère). Une échelle de cotation de 0 à 9 a été utilisée pour évaluer les sensations de prurit et de brûlure (0 = absent, 1-2-3 = léger, 4-5-6 = modéré et 7-8-9 = sévère).
Des mesures de cornéométrie, de colorimétrie et de pH ont été effectuées à J0, J14 et J21 ; ainsi que des mesures de Perte Insensible en Eau (PIE). Toutes ces mesures ont été réalisées avant l'application des produits après une période d'acclimatation de 15 minutes pour tous les sujets.

### Critères d'évaluation :

### Critère principal :

- Evaluation clinique des signes d'irritation : score total érythème et desquamation sur une échelle de cotation de 0 à 6.
- Evaluation clinique des symptômes d'irritation : score total prurit et brûlure échelle de cotation de 0 à 18.
- Tolérance.

### Critères secondaires :

- Mesures de colorimétrie paramètre a* (variable de chromaticité, axe rouge-vert).
- Mesures biophysiques : cornéométrie (capacitance électrique en Unité Arbitraire), pH et PIE (en g/m²/h).

### Analyses statistiques :

Pour le test de tolérance, une aire sous la courbe a été calculée si appropriée, pour quantifier un effet global des traitements sur la période d'évaluation. Les paramètres ont été au préalable ajustés par leur valeur initiale avant application. La variable PIE a été transformée en logarithme naturel afin de normaliser la distribution et de stabiliser la variance.

Une analyse de variance comprenant les facteurs sujet, zone, produit suivi de test t de student a été utilisée pour comparer les AUC (le seuil de 0.05 a été utilisé pour conclure à une significativité).

### Résultats :

### Critères principaux :

### Signes et symptômes d'irritation :

Les scores d'irritation étaient souvent nuls. Aucune analyse statistique n'a donc été réalisée. Seul un sujet a présenté un score non nul. Les cotations étaient cependant très proches de 0 (score total de 1).

### Score total sur les 4 visites :

| | Spray D | Spray E | Zone Non Traitée |
|---|---|---|---|
| Erythème | 0 | 0 | 0 |
| Desquamation | 0 | | 0 |
| Prurit | 0 | 0 | 0 |
| Brûlure | 1 | 0 | 0 |

### Tolérance :

La tolérance a été suivie au travers des évènements indésirables (El). 9 sujets (75.0%) sur 12 ont présenté un El Ces El n'étant pas liés au traitement, la tolérance aux produits a été jugée très bonne.

### Critères secondaires :

### Evolution du paramètre a* : colorimétrie (n = 12 ; moyenne ±sem)

| | J0 | J14 | J21 |
|---|---|---|---|
| Spray D | 8.38±0.41 | 8.73±0.43 | 8.81±0.45 |
| Spray E | 8.48±0.36 | 8.08±0.38 | 8.45±0.43 |
| Zone non traitée | 7.71±0.54 | 7.86±0.56 | 7.96±0.53 |

Il n'y a pas de différence significative entre les deux formulations testées pour le paramètre a* qui permette d'évaluer plus précisément l'érythème et donc l'irritation. Ceci est à relier à l'absence de pouvoir irritant observé sur le plan clinique.

### Evolution de la PIE (n = 12 : moyenne ±sem)

| | J0 | J14 | J21 |
|---|---|---|---|
| Spray D | 63.64±2.26 | 59.69 ±1.58 | 61.58 ±1.61 |
| Spray E | 61.33 ±2.06 | 62.42 ±1.87 | 64.61 ±2.39 |
| Zone non traitée | 60.39 ±1.99 | 59.78 ±2.08 | 61.72 ±2.06 |

Les deux formulations testées n'ont pas d'effets significatifs sur la Perte Insensible en Eau (PIE), qui traduit le niveau d'intégrité fonctionnelle de la couche cornée. Ceci confirme l'absence de potentiel irritant des deux formulations.

### Evolution du pH (n = 12 ; moyenne ±sem)

| | J0 | J14 | J21 |
|---|---|---|---|
| Spray D | 5.34±0.18 | 4.98 ±0.20 | 4.96 ±0.09 |
| Spray E | 5.29 ±0.17 | 4.98 ±0.13 | 5.04±0.16 |
| Zone non traitée | 5.17 ±0.22 | 4.98 ±0.14 | 4.91 ±0.11 |

Il n'y a pas de différence significative entre les deux formulations testées pour le pH. Elles préservent donc le pH physiologique acide cutané, qui est un des éléments de la fonction barrière de la peau.

**Conclusion** : Cette étude montre la bonne tolérance des véhicules spray malgré la forte teneur en éthanol de 30%.

## Revendications

1. Composition comprenant, dans un véhicule alcoolique pharmaceutiquement acceptable et solubilisant de l'actif ;
a) une quantité thérapeutiquement efficace d'au moins un actif pharmaceutique qui est le 17-propionate de clobétasol,
b) au moins un silicone volatile,
c) une phase huileuse non volatile,
ladite composition étant pulvérisable.

2. Composition selon la revendication 1, **caractérisée en ce qu'**elle comprend :
a) Entre 0,000 et 20 % en poids de l'actif,
b) Entre 25 et 95 % en poids de silicone volatile,
c) Entre 1 et 50 % en poids de phase huileuse non volatile.

3. Composition selon l'une des revendications 1 ou 2, **caractérisée en ce qu'**elle contient entre 0.01 % à 2% en poids de 17-propionate de clobétasol,

4. Composition selon l'une des revendications 1 à 3, **caractérisée en ce que** le silicone volatile est choisi parmi le groupe constitué par les polydimethylcyclosiloxanes et les polysiloxanes linéaires de bas poids moléculaires.

5. Composition selon l'une des revendications 1 à 4, **caractérisée en ce que** le silicone volatile est un polysiloxane linéaire du type hexaméthyldisiloxane.

6. Composition selon l'une des revendications 1 à 5, **caractérisée en ce qu'**elle contient entre 55 et 65% en poids d'hexaméthyldisiloxane.

7. Composition selon l'une des revendications 1 à 6, **caractérisée en ce que** la phase huileuse non volatile est l'huile de paraffine,

8. Composition selon l'une des revendications 1 à 7. **caractérisée en ce qu'**elle contient entre 5 et 15% d'huile de paraffine.

9. Composition selon l'une quelconque des revendications précédentes, **caractérisée en ce qu'**elle contient également une gomme de silicone.

10. composition selon l'une quelconque des revendications 1 à 9, **caractérisée en ce qu'**elle a une viscosité inférieure à 50 centistokes.

11. Composition selon l'une quelconque des revendications 1 à 10, **caractérisée en ce qu'**elle contient :
a) 0.05% de 17-propionate de clobétasol
b) 60% d' hexaméthyldisiloxane,
c) 10% d'huile de paraffine,
d) 29.95% d'éthanol.

12. Composition selon l'une quelconque des revendications 1 à 10, **caractérisée en ce qu'**elle contient ;
a) 0.05% de 17-propionate de clobétasol
b) 59.4% d'hexaméthyldisiloxane,
c) 0.6% de gomme de silicone,
d) 10% d'huile de paraffine,
e) 29.95% d'éthanol,

13. Utilisation d'une composition selon l'une quelconque des revendications 1 à 12 pour la fabrication d'un médicament destiné au traitement :
des affections dermatologiques liées à un désordre de la kératinisation portant sur la différenciation et sur la prolifération notamment les acnés vulgaires, comédoniennes polymorphes, rosacées, les acnés nodulokystiques, conglobata, les acnés séniles, les acnés secondaires telles que l'acné solaire, médicamenteuse ou professionnelle,
- des ichtyoses, des états ichtyosiformes de la maladie de Darrier, des kératadermies palmoplantaires, des leucoplasies et des états leucoplasiformes, du lichen cutané ou muqueux (buccal).
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma, l'atopie respiratoire ou l'hypertrophie gingivale,
- des proliférations dermiques ou épidermiques bénignes ou malignes, d'origine virale ou non, notamment les verrues vulgaires, les verrues planes l'épidermodysplasie verruciforme, les papillomatoses orales ou florides, le lymphome T,
- des proliférations pouvant être induites par les ultra-violets notamment des épithélioma baso et spinocellulaires,
- des lésions précancéreuses cutanées notamment les kératoacanthomes,
- des dermatoses immunes notamment le lupus érythémateux,
- des maladies immunes bulleuses,
- des maladies du collagène notamment la sclérodermie,
- des affections dermatologiques ou générales à composante immunologique,
- de désordres cutanés dus à une exposition aux rayonnements U.V, du vieillissement de la peau, photo-induit ou chronologique ou des pigmentations et des kératoses actiniques, ou toutes pathologies associées au vieillissement chronologique ou actinique notamment la xérose,
- des troubles de la fonction sébacée notamment l'hyperséborrhée de l'acné, la séborrhée simple ou la dermite séborrhéique,
des troubles de la cicatrisation ou des vergetures,
- des désordres de la pigmentation, tel l'hyperpigmentation, le mélasma, l'hypopigmentation ou le vitiligo,
- des affections du métabolisme des lipides, tel l'obésité, l'hyperlipidémie, le diabète non insulino-dépendant ou le syndrome X,
- des affections inflammatoires telles que l'arthrite,
- des états cancéreux ou précancéreux,
- -de l'alopécie de différentes origines, notamment l'alopécie due à la chimiothérapie ou aux rayonnements,
- des troubles du systèmes immunitaire, tel l'asthme, le diabète sucré de type I, la sclérose en plaque, ou autres disfonctionnements sélectifs du système immunitaire, ou
- des affections du système cardiovasculaire telles que l'artériosclérose ou l'hypertension.

14. Utilisation d'une composition, selon la revendication 13, pour le traitement du psoriasis.

15. Procédé d'amélioration de la pénétration d'un actif pharmaceutique **caractérisé en ce que** l'on applique sur la peau une composition
selon l'une des revendications 1 à 12.
ladite composition étant appliquée par pulvérisation.

16. Procédé selon la revendication 15, **caractérisé en ce que** le silicone volatile est l'hexaméthyldisiloxane, la phase huileuse non volatile est l'huile de paraffine.

17. Procédé selon l'une des revendications 15 ou 16 **caractérisé en ce que** la composition comprend également une gomme de silicone.

## Claims

1. Composition comprising, in a pharmaceutically acceptable alcoholic vehicle that can solubilize the active agent:
a) a therapeutically effective amount of at least one pharmaceutical active agent which is clobetasol 17-propionate,
b) at least one volatile silicone,
c) a nonvolatile oily phase, said composition being sprayable.

2. Composition according to Claim 1, **characterized in that** it comprises:
a) between 0.0001 and 20% by weight of the active agent,
b) between 25 and 95% by weight of volatile silicone,
c) between 1 and 50% by weight of nonvolatile oily phase.

3. Composition according to either of Claims 1 and 2, **characterized in that** it contains between 0.01% and 2% by weight of clobetasol 17-propionate.

4. Composition according to one of Claims 1 to 3, **characterized in that** the volatile silicone is chosen from the group consisting of polydimethylcyclosiloxanes and low molecular weight linear polysiloxanes.

5. Composition according to one of Claims 1 to 4, **characterized in that** the volatile silicone is a linear polysiloxane of the hexamethyldisiloxane type.

6. Composition according to one of Claims 1 to 5, **characterized in that** it contains between 55 and 65% by weight of hexamethyldisiloxane.

7. Composition according to one of Claims 1 to 6, **characterized in that** the nonvolatile oily phase is paraffin oil.

8. Composition according to one of Claims 1 to 7, **characterized in that** it contains between 5 and 15% of paraffin oil.

9. Composition according to any one of the preceding claims, **characterized in that** it also contains a silicone gum.

10. Composition according to any one of Claims 1 to 9, **characterized in that** it has a viscosity of less than 50 centistokes.

11. Composition according to any one of Claims 1 to 10, **characterized in that** it contains:
a) 0.05% of clobetasol 17-propionate,
b) 60% of hexamethyldisiloxane,
c) 10% of paraffin oil,
d) 29.95% of ethanol.

12. Composition according to any one of Claims 1 to 10, **characterized in that** it contains:
a) 0.05% of clobetasol 17-propionate,
b) 59.4% of hexamethyldisiloxane,
c) 0.6% of silicone gum,
d) 10% of paraffin oil,
e) 29.95% of ethanol.

13. Use of a composition according to any one of Claims 1 to 12, for producing a medicinal product intended for treating:
- dermatological conditions associated with a keratinization disorder relating to differentiation and to proliferation, in particular common acne, comedo-type acne, polymorphic acne, rosacea, nodulocystic acne, acne conglobata, senile acne, and secondary acne such as solar, drug-related or occupational acne,
- ichthyoses, ichthyosiform conditions, Darrier's disease, palmoplantar keratoderma, leukoplakia and leukoplakiform conditions, and cutaneous or mucosal (oral) lichen,
- dermatological conditions with an inflammatory immunoallergic component, with or without a cell proliferation disorder, in particular cutaneous, mucosal or ungual psoriasis, psoriatic rheumatism, cutaneous atopy, such as eczema, respiratory atopy or gingival hypertrophy,
- benign or malignant dermal or epidermal proliferations, of viral or nonviral origin, in particular common warts, flat warts, epidermodysplasia verruciformis, oral or florid papillomatoses, and T lymphoma,
- proliferations which may be induced by ultraviolet light, in particular basal cell epithelioma and spinocellular epithelioma,
- precancerous skin lesions, in particular keratoacanthomas,
- immune dermatoses, in particular lupus erythematous,
- bullous immune diseases,
- collagen diseases, in particular scleroderma,
- dermatological or systemic conditions with an immunological component,
- skin disorders due to exposure to UV radiation, or light-induced or chronological ageing of the skin, or actinic keratoses and pigmentations, or any pathologies associated with chronological or actinic ageing, in particular xerosis,
- sebaceous function disorders, in particular acne-related hyperseborrhoea or simple seborrhoea or seborrhoeic dermatitis,
- cicatrization disorders or stretch marks,
- pigmentation disorders, such as hyperpigmentation, melasma, hypopigmentation or vitiligo,
- lipid metabolism conditions, such as obesity, hyperlipidemia, non-insulin-dependent diabetes or syndrome X,
- inflammatory conditions such as arthritis,
- cancerous or precancerous states,
- alopecia of various origins, in particular alopecia caused by chemotherapy or radiation,
- immune system disorders, such as asthma, type 1 diabetes mellitus, multiple sclerosis or other selective dysfunctions of the immune system, or
- cardiovascular system conditions such as arteriosclerosis or hypertension.

14. Use of a composition, according to Claim 13, for treating psoriasis.

15. Process for improving the penetration of a pharmaceutical active agent, **characterized in that** a composition according to one of Claims 1 to 12 is applied to the skin, said composition being applied by spraying.

16. Process according to Claim 15, **characterized in that** the volatile silicone is hexamethyldisiloxane, and the nonvolatile oily phase is paraffin oil.

17. Process according to either of Claims 15 and 16, **characterized in that** the composition also comprises a silicone gum.

## Patentansprüche

1. Zusammensetzung, die in einem pharmazeutisch akzeptablen, alkoholischen und den Wirkstoff solubilisierenden Träger enthält:
a) eine therapeutisch wirksame Menge mindestens eines pharmazeutischen Wirkstoffes, bei dem es sich um das Clobetasol-17-propionat handelt;
b) mindestens ein flüchtiges Silicon;
c) mindestens eine nichtflüchtige Ölphase;
wobei die Zusammensetzung zerstäubbar ist.

2. Zusammensetzung nach Anspruch 1, **dadurch gekennzeichnet, dass** sie enthält:
a) 0,0001 bis 20 Gew.-% Wirkstoff;
b) 25 bis 95 Gew.-% flüchtiges Silicon;
c) 1 bis 50 Gew.-% nichtflüchtige Ölphase.

3. Zusammensetzung nach einem der Ansprüche 1 oder 2, **dadurch gekennzeichnet, dass** sie 0,001 bis 2 Gew.-% Clobetasol-17-propionat enthält.

4. Zusammensetzung nach einem der Ansprüche 1 bis 3, **dadurch gekennzeichnet, dass** das flüchtige Silicon unter den Polydimethylcyclosiloxanen und den linearen Polysiloxanen mit niedrigen Molmassen ausgewählt ist.

5. Zusammensetzung nach einem der Ansprüche 1 bis 4, **dadurch gekennzeichnet, dass** das flüchtige Silicon ein lineares Polysiloxan vom Typ Hexamethyldisiloxan ist.

6. Zusammensetzung nach einem der Ansprüche 1 bis 5, **dadurch gekennzeichnet, dass** sie 55 bis 65 Gew.-% Hexamethyldisiloxan enthält.

7. Zusammensetzung nach einem der Ansprüche 1 bis 6, **dadurch gekennzeichnet, dass** es sich bei der nichtflüchtigen Ölphase um Paraffinöl handelt.

8. Zusammensetzung nach einem der Ansprüche 1 bis 7, **dadurch gekennzeichnet, dass** sie 5 bis 15 Gew,-% Paraffinöl enthält.

9. Zusammensetzung nach einem der vorhergehenden Ansprüche, **dadurch gekennzeichnet, dass** sie ferner einen Silicongummi enthält.

10. Zusammensetzung nach einem der Ansprüche 1 bis 9, **dadurch gekennzeichnet, dass** sie eine Viskosität unter 50 Centistokes besitzt.

11. Zusammensetzung nach einem der Ansprüche 1 bis 10, **dadurch gekennzeichnet, dass** sie enthält:
a) 0,05 % Clobetasol-17-propionat;
b) 60 % Hexamethyldisiloxan;
c) 10 % Paraffinöl;
d) 29,95 % Ethanol.

12. Zusammensetzung nach einem der Ansprüche 1 bis 10, **dadurch gekennzeichnet, dass** sie enthält:
a) 0,05 % Clobetasol-17-propionat;
b) 59,4 % Hexamethyldisiloxan;
c) 0,6 % Silicongummi
d) 10 % Paraffinöl;
e) 29,95 % Ethanol.

13. Verwendung einer Zusammensetzung nach einem der Ansprüche 1 bis 12 für die Herstellung eines Arzneimittels, das für die Behandlung der folgenden Erkrankungen vorgesehen ist:
- dermatologischen Erkrankungen, die mit einer Störung der Keratinisierung in Zusammenhang stehen, die auf der Differenzierung und/oder Proliferation beruht, insbesondere zur Behandlung von Acne vulgaris, Acne comedonica, polymorpher Akne, Acne rosacea, nodulocystischer Akne, Acne conglobata, Acne senilis, sekundären Akneformen, wie Acne solaris, Acne medicamentosa oder Acne professionalis,
- Ichthyosen, ichthyosiformen Zuständen, der Darier-Krankheit, Palmoplantarkeratosen, Leukoplasien und leukoplasiformen Zuständen und Lichen der Haut oder der Schleimhaut (buccal),
- dermatologischen Erkrankungen mit einer entzündlichen immunoallergischen Komponente, mit oder ohne Störung der Zellproliferation, insbesondere Psoriasis der Haut, der Schleimhäute oder der Nägel, Psoriasis arthropathica, Atopie der Haut, wie Ekzemen, respiratorischer Atopie oder Hypertrophie des Zahnfleisches,
- Proliferationen der Dermis oder Epidermis, die benigne oder maligne und gegebenenfalls viralen Ursprungs sein können, insbesondere Verruca vulgaris, Verruca plana und Epidermodysplasia verruciformis, oralen oder floriden Papillomatosen, T-Lymphom,
- Proliferationen, die durch UV-Strahlung induziert sein können, insbesondere Epithelioma basocellulare und spinocellulare,
- präkanzerösen Hautläsionen, insbesondere Keratoakanthomen,
- Immundermatosen, insbesondere Lupus erythematodes,
- bullösen Immunerkrankungen;
- Collagenerkrankungen, insbesondere Sklerodermie,
- dermatologischen oder allgemeinen Erkrankungen mit immunologischer Komponente,
- Hautstörungen, die durch eine UV-Exposition verursacht werden, Hautalterung, die lichtinduziert oder altersbedingt sein kann, oder Pigmentierungen und aktinischen Keratosen oder allen anderen Erkrankungen, die mit; der altersbedingten oder aktinischen Alterung zusammenhängen, insbesondere Xerosis,
- Funktionsstörungen der Talgdrüsen, insbesondere Hyperseborrhoe bei Akne, Seborrhoe simplex oder seborrhoische Dermatitis,
- Störungen der Wundheilung oder Dehnungsstreifen,
- Pigmentierungsstörungen, wie Hyperpigmentierung, Melasma, Hypopigmentierung oder Vitiligo,
- Störungen des Lipidstoffwechsels, wie Adipositas, Hyperlipidämie, nichtinsulinpflichtigem Diabetes oder X-Syndrom,
- entzündlichen Erkrankungen, wie Arthritis,
- kanzerösen oder präkanzerösen Zuständen,
- Alopezie unterschiedlicher Ursache, insbesondere durch Chemotherapie oder Bestrahlung hervorgerufener Alopezie,
- Störungen des Immunsystems, wie Asthma, Diabetes Typ I, multipler Sklerose oder anderen selektiven Funktionsstörungen des Immunsystems, oder
- Erkrankungen des kardiovaskulären Systems, wie Arteriosklerose oder Bluthochdruck.

14. Verwendung einer Zusammensetzung nach Anspruch 13 für die Behandlung von Psoriasis.

15. Verfahren zur Verbesserung der Penetration eines pharmazeutischen Wirkstoffes in die Haut, **dadurch gekennzeichnet, dass** auf die Haut eine Zusammensetzung nach einem der Ansprüche 1 bis 12 aufgebracht wird, wobei die Zusammensetzung durch Zerstäuben appliziert wird.

16. Verfahren nach Anspruch 15, **dadurch gekennzeichnet, dass** es sich bei dem flüchtigen Silicon um Hexamethydisiloxan und bei der nichtflüchtigen Ölphase um Paraffinöl handelt.

17. Verfahren nach einem der Ansprüche 15 oder 16, **dadurch gekennzeichnet, dass** die Zusammensetzung ferner einen Silicongummi enthält.
